# EUROPÄISCHE PATENTSCHRIFT

(11) **EP 0 601 138 B1**
(45) Veröffentlichungstag und Bekanntmachung des Hinweises auf die Patenterteilung: **18.09.1996**
(21) Anmeldenummer: 93906573.6
(22) Anmeldetag: 20.03.1993
(51) Int. Cl.: C07C 205/37, C07C 201/12, C07C 205/12, C07C 201/08

(54) **VERFAHREN ZUR HERSTELLUNG VON 5-ALKOXY-2,4-DINITROALKYLBENZOLEN**
METHOD OF PREPARING 5-ALKOXY-2,4-DINITROALKYLBENZENE COMPOUNDS
PROCEDE DE FABRICATION DE 5-ALCOXY-2,4-DINITROALKYLBENZENES

(30) Priorität: 17.06.1992 DE 4219755
(43) Veröffentlichungstag der Anmeldung: 15.06.1994
(73) Patentinhaber: Wella Aktiengesellschaft, D-64295 Darmstadt (DE)
(72) Erfinder: BALZER, Wolfgang, R., D-64665 Alsbach (DE); CLAUSEN, Thomas, D-64665 Alsbach (DE); WEINGES, Alexa, D-69121 Heidelberg (DE)
(86) Internationale Anmeldenummer: EP9300678
(87) Internationale Veröffentlichungsnummer: WO9325512

(56) Entgegenhaltungen:
- EP-A- 0 204 111
- EP-A- 0 252 351
- WO-A-92/04005
- JOURNAL OF THE CHEMICAL SOCIETY, PERKIN TRANSACTIONS 2 1972, LETCHWORTH GB Seiten 999 - 1005 J.F. CORBETT 'Benzoquinone Imines. Part X. The Mechanism and Kinetics of the Reactions of p-Benzoquinone Di-imine and p-Benzoquinone Moniomine with C-Methoxy-m-diamines and p-Methoxy- and p-Chloro-phenols.' in der Anmeldung erwähnt

## Beschreibung

Gegenstand der vorliegenden Erfindung ist ein Verfahren zur Herstellung von 5-Alkoxy-2,4-dinitro-alkylbenzolen der allgemeinen Formel (I) 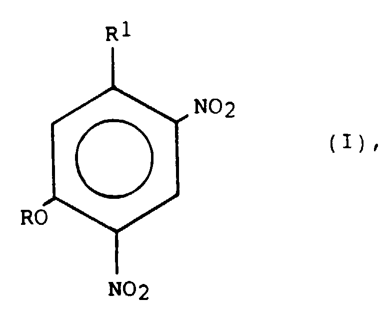 worin R¹ ein verzweigter oder unverzweigter C₁-bis C₄-Alkylrest ist und R einen verzweigten oder unverzweigten C₁- bis C₆-Alkylrest, einen C₂- bis C₄-Hydroxyalkylrest oder einen C₃- bis C₄-Dihydroxyalkylrest darstellt, welche unter anderem als Farbstoffvorstufe, zum Beispiel für Haarfarbstoffe, verwendet werden können.

Es sind aus der Literatur bereits mehrere verfahren zur Herstellung von 5-Alkoxy-2,4-dinitro-alkylbenzolen der Formel (I) bekannt. Diese Verfahren sind jedoch in vielerlei Hinsicht unbefriedigend.

So wurde beispielsweise von J.F. Corbett im Journal of the Chemical Society Perkin II, Seite 999 (1972) ein Verfahren zur Herstellung von 5-Methoxy-2,4-dinitrotoluol beschrieben, bei dem man 5-Chlor-2,4-dinitrotoluol und Methanol in Gegenwart von Kaliumhydroxid unter Rückfluß erwärmt. Die Ausbeute bei diesem Verfahren ist jedoch nicht befriedigend.

Weiterhin wird in der DE-OS 3 622 784 ein Verfahren zur Herstellung von 5-Alkoxy-2,4-dinitro-alkylbenzolen beschrieben, wobei 5-Alkyl-2,4-dinitro-phenol in einem geeigneten Lösungsmittel mit einem entsprechenden Alkylhalogenid in Gegenwart einer Base umgesetzt wird und anschließend das Produkt durch Zugabe von Wasser ausgefällt wird. Obwohl die Alkylierung nach diesem Verfahren in befriedigender Ausbeute abläuft (50 - 77 %) birgt der gesamte Herstellungsweg doch einige Nachteile in sich. So muß das hierfür benötigte Ausgangsmaterial durch eine dreistufige Synthese ausgehend von 3-Methyl-6-nitrophenol über Einführung einer Mesylschutzgruppe, Nitrierung und anschließender Spaltung der Schutzgruppe mit einer Gesamtausbeute von etwa 76 % hergestellt werden. Weiterhin müssen für die Darstellung der Alkylverbindungen die nicht unbedenklichen Alkylhalogenide als Reagenzien verwendet werden. Das vorstehend beschriebene Verfahren zur Herstellung von 5-Alkoxy-2,4-dinitroalkylbenzolen der allgemeinen Formel (I) ist zwar technisch mit einer befriedigenden Ausbeute durchführbar, besitzt aber Nachteile gerade in ökonomischer und ökologischer Hinsicht. Durch die Vielzahl der benötigten Reaktionsschritte und auch die Art der verwendeten Reagenzien wird bei diesem Verfahren ein großer Energieeinsatz zur Durchführung der Reaktionen benötigt. Zudem fallen bei jeder Reaktionsstufe Abfallprodukte an (Nebenbestandteile und Lösungsmittel), die - um eine zusätzliche Umweltbelastung zu vermeiden - mit großem technischen Aufwand aufgearbeitet und entsorgt werden müssen. Diese Nachteile beinhaltet auch in großem Maße das beschriebene Herstellungsverfahren von Corbett, bei dem der Anteil an Nebenprodukten größer ist als der Anteil der gewünschten Reaktionsprodukte.

Es bestand daher die Aufgabe, ein Verfahren zur Herstellung von 5-Alkoxy-2,4-dinitro-alkylbenzolen der Formel (I) zur Verfügung zu stellen, das eine einfache und wirtschaftliche Herstellung dieser Verbindungen ermöglicht und bei dem die vorstehend aufgeführten Nachteile vermieden werden.

Überraschenderweise wurde nunmehr gefunden, daß die gestellte Aufgabe in hervorragender Weise gelöst wird, indem man die 5-Alkoxy-2,4-dinitro-alkylbersole der allgemeinen Formel (I) bei niedrigen Temperaturen durch nucleophile Substitution von 2,4-Dinitro-5-fluor-alkylbenzolen herstellt.

Gegenstand der vorliegenden Erfindung ist daher ein Verfahren zur Herstellung von 5-Alkoxy-2,4-dinitroalkylbenzolen, der allgemeinen Formel (I) 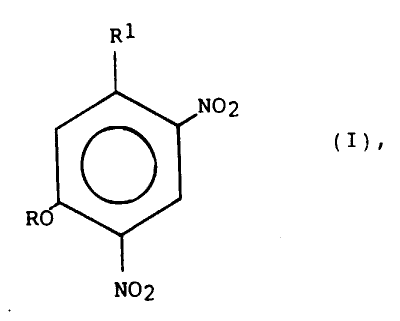 wobei R¹ einen verzweigten oder unverzweigten C₁-bis C₄-Alkylrest bedeutet und R ein verzweigter oder unverzweigter C₁- bis C₆-Alkylrest, ein C₂- bis C₄-Hydroxyalkylrest oder ein C₃- bis C₄-Dihydroxyalkylrest ist, welches dadurch gekennzeichnet ist, daß man gemäß dem nachfolgenden allgemeinen Reaktionsschema ein 3-Fluor-alkylbenzol (II) nitriert und das erhaltene 2,4-Dinitro-5-fluor-alkylbenzol (III) unter Zusatz von Natriumhydroxid oder Kaliumhydroxid mit einem geeigneten Alkohol bei -5°C bis +25°C umsetzt.

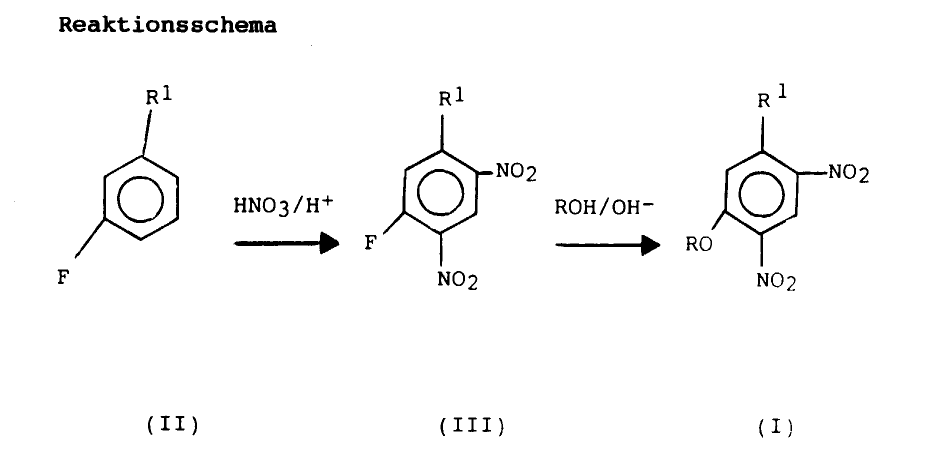

Bei dem erfindungsgemäßen Verfahren wird zunächst ein 3-Fluor-alkylbenzol der allgemeinen Formel (II) gemäß der aus der DE-OS 40 28 661 bekannten Vorschrift nitriert. Anschließend wird das erhaltene 2,4-Dinitro-5-fluor-alkylbenzol der Formel (III) unter kräftigem Rühren mit einem geeigneten Alkohol sowie Natriumhydroxid oder Kaliumhydroxid bei einer Temperatur von -5°C bis +25°C, vorzugsweise bei einer Temperatur von 0°C bis +10°C, umgesetzt, wobei der Alkohol gleichzeitig als Reagenz und Lösungsmittel dient und deshalb in einem vielfachen molaren Überschuß eingesetzt wird.

Als Alkohol können sowohl Monohydroxyalkylverbindungen mit 1 bis 4 Kohlenstoffatomen in der Alkylkette als auch Dihydroxyalkylverbindungen mit 2 bis 4 Koklenstuffatomen in der Alkylkette oder Trihydroxyalkylverbindungen mit 3 oder 4 Kohlenstoffatomen in der Alkylkette verwendet werden, wobei die Verwendung von Methanol, Ethanol und Ethylenglykol besonders bevorzugt ist.

Das Natriumhydroxid oder Kaliumhydroxid kann sowohl in einer im Vergleich zum 2,4-Dinitro-5-fluor-alkylbenzol äquimolaren Menge als auch in einem molaren Überschuß eingesetzt werden, wobei ein molarer Überschuß an Natriumhydroxid oder Kaliumhydroxid bevorzugt ist.

Besonders bevorzugt ist ein molares Verhältnis von 2,4-Dinitro-5-fluor-alkylbenzol zu Natriumhydroxid oder Kaliumhydroxid von 1 : 1,4.

Als 3-Fluor-alkylbenzol der Formel (II)wird vorzugsweise 3-Fluor-toluol eingesetzt, aus dem durch Nitrierung 2,4-Dinitro-5-fluor-toluol erhalten wird.

Bei der vorstehend beschriebenen Versuchsdurchführung wird das 5-Alkoxy-2,4-dinitro-alkylbenzol der Formel (I) in guten Ausbeuten erhalten, während bei Verwendung von 2,4-Dinitro-5-chlor-alkylbenzolen oder 2,4-Dinitro-5-brom-alkylbenzolen anstelle der erfindungsgemäßen 2,4-Dinitro-5-fluor-alkylbenzole die Verbindungen der Formel (I) nur in geringen Ausbeuten oder aber in stark verunreinigter Form erhalten werden.

Eine Durchführung des erfindungsgemäßen Verfahrens bei höheren als den angegebenen Temperaturen führt überraschenderweise ebenfalls zu einer deutlichen Verschlechterung der Ausbeute. So wird die Ausbeute um ein Viertel bis zwei Drittel reduziert, wenn die Umsetzung des 2,4-Dinitro-5-fluor-alkylbenzols mit dem Alkohol nicht bei -5°C bis 25°C sondern bei höhieren Temperaturen (beispielsweise unter Rückfluß) durchgeführt wird.

Die nachfolgenden Beispiele sollen den Gegenstand der Erfindung näher erläutern, ohne den Gegenstand der Erfindung auf diese Beispiele zu beschränken.

### Beispiele

### Beispiel 1 5-Methoxy-2,4-dinitro-toluol

Jeweils 2,8 g (14 mmol) 2,4-Dinitro-5-fluor-toluol, welches durch Nitrierung von 3-Fluortoluol gemäß Beispiel A, Stufe 1 der DE-OS 40 28 661 hergestellt wurde, werden in 100 ml Methanol gelöst und unter kräftigem Rühren bei verschiedenen Temperaturen mit einer Mischung von 1,12 g (20 mmol) Kaliumhydroxidpulver und 20 ml Methanol langsam versetzt. Anschließend wird die Reaktionsmischung auf Wasser gegossen, der erhaltene Niederschlag abfiltriert und aus Isopropanol umkristallisiert.

Der Schmelzpunkt beträgt 101°C.

In Abhängigkeit von der Reaktionstemperatur werden folgende Ausbeuten erhalten:

| Reaktionstemperatur | Ausbeute % der Theorie |
|---|---|
| Rückfluß (> 65°C) | 38 |
| Raumtemperatur (20-25°C) | 51 |
| Eiskülung (0-10°C) | 71 |

### Beispiel 2 5-Ethoxy-2,4-dinitro-toluol

Jeweils 2,8 g (14 mmol) 2,4-Dinitro-5-fluor-toluol, das durch Nitrierung von 3-Fluor-toluol gemäß Beispiel A, Stufe 1 der DE-OS 40 28 661 hergestellt wurde, werden in 100 ml Ethanol gelöst und unter kräftigem Rühren bei verschiedenen Temperaturen mit einer Mischung von 1,12 g (20 mmol) Kaliumhydroxidpulver und 20 ml Ethanol langsam versetzt. Anschließend wird die Reaktionsmischung auf Wasser gegossen, der erhaltene Niederschlag abfiltriert und aus Isopropanol umkristallisiert.

Der Schmelzpunkt beträgt 95 bis 96°C.

In Abhängigkeit von der Reaktionstemperatur werden folgende Ausbeuten erhalten:

| Reaktionstemperatur | Ausbeute % der Theorie |
|---|---|
| Rückfluß (etwa 80°C) | 25 |
| Raumtemperatur (20-25°C) | 39 |
| Eiskülung (0-10°C) | 76 |

Alle in der vorliegenden Patentanmeldung angegebenen Prozentzahlen stellen, falls nicht anders angegeben, Gewichtsprozente dar.

## Patentansprüche

1. Verfahren zur Herstellung von 5-Alkoxy-2,4-dinitroalkylbenzolen der allgemeinen Formel (I) 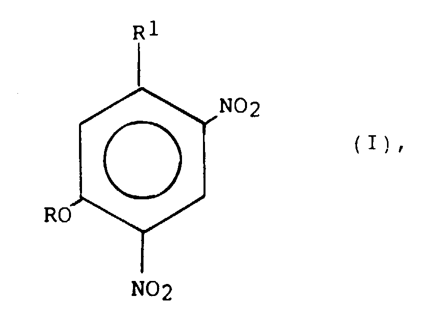 wobei R¹ einen verzweigten oder unverzweigten C₁-bis C₄-Alkylrest bedeutet und R ein verzweigter oder unverzweigter C₁- bis C₆-Alkylrest, ein C₂-bis C4-Hydroxyalkylrest oder ein C₃- bis C₄-Di-hydroxyalkylrest ist, dadurch gekennzeichnet, daß man ein 3-Fluor-alkylbenzol nitriert und das erhaltene 2,4-Dinitro-5-fluor-alkylbenzol anschließend unter Zusatz von Natriumhydroxid oder Kaliumhydroxid mit einem geeigneten Alkohol bei -5°C bis +25°C umsetzt.

2. Verfahren nach Anspruch 1, dadurch gekennzeichnet, daß die Umsetzung des 2,4-Dinitro-5-fluor-alkylbenzols mit dem Alkohol bei 0°C bis +10°C erfolgt.

3. Verfahren nach Anspruch 1 oder 2, dadurch gekennzeichnet, daß als 2,4-Dinitro-5-fluor-alkylbenzol ein 2,4-Dinitro-5-fluor-toluol verwendet wird.

4. Verfahren nach einem der Ansprüche 1 bis 3, dadurch gekennzeichnet, daß der Alkohol ausgewählt ist aus Methanol, Ethanol und Ethylenglykol.

5. Verfahren nach einem der Ansprüche 1 bis 3, dadurch gekennzeichnet, daß das molare Verhältnis von 2,4-Dinitro-5-fluor-alkylbenzol zu Natriumhiydroxid oder Kaliumhydroxid gleich 1 : 1,4 ist.

## Claims

1. Method of preparing 5-alkoxy-2,4-dinitroalkylbenzenes of the general formula (I), 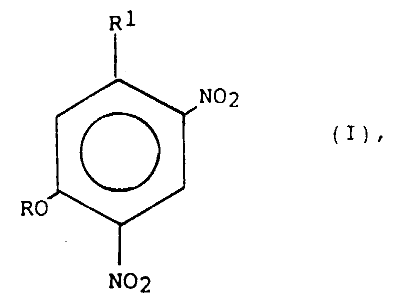 wherein R¹ is a branched or unbranched C₁ to C₄ alkyl residue and R is a branched or unbranched C₁ to C₆ alkyl residue, a C₂ to C₄ hydroxy alkyl residue or a C₃ to C₄ dihydroxy alkyl residue, characterised in that a 3-fluoro-alkylbenzene is nitrated and the resultant 2,4-dinitro-5-fluoroalkylbenzene is subsequently reacted with a suitable alcohol at from -5°C to +25°C with the addition of sodium hydroxide or potassium hydroxide.

2. Method according to Claim 1,
characterised in that the 2,4-dinitro-5-fluoroalkylbenzene is reacted with alcohol at from 0°C to +10°C.

3. Method according to Claim 1 or 2,
characterised in that a 2,4-dinitro-5-fluorotoluene is used as the 2,4-dinitro-5-fluoroalkylbenzene.

4. Method according to any one of Claims 1 to 3, characterised in that the alcohol is selected from methanol, ethanol and ethylene glycol.

5. Method according to any one of Claims 1 to 3, characterised in that the molar ratio of 2,4-dinitro-5-fluoro-alkylbenzene to sodium hydroxide or potassium hydroxide is 1:1.4.

## Revendications

1. Procédé de préparation des 5-alcoxy-2,4-dinitro-alkyl-benzène de formule générale (I) 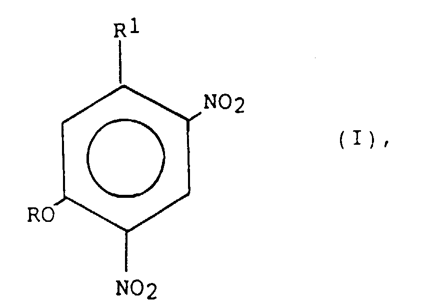 dans laquelle R¹ représente un reste alkyle en C₁-C₄ ramifié ou non ramifié, et R un reste alkyle en C₁ à C₆, ramifié ou non ramifié, un reste hydroxyalkyle en C₂ à C₄, ou un reste dihydroxyalkyle en C₃-C₄, caractérisé en ce qu'on effectue une nitration d'un 3-fluoro-alkyl-benzène, et qu'on fait réagir ensuite le 2,4-dinitro-5-fluoro-alkyl-benzène obtenu par addition d'hydroxyde de sodium ou d'hydroxyde de potassium avec un alcool approprié à une température de -5°C à +25°C.

2. Procédé selon la revendication 1, caractérisé en ce que la réaction des 2,4-dinitro-5-fluoro-alkylbenzène avec l'alcool se produit entre 0°C et +10°C.

3. Procédé selon la revendication 1 ou 2, caractérisé en ce que on utilise comme 2,4-dinitro-5-fluoro-alkylbenzène,un 2,4-dinitro-5-fluoro-toluène.

4. Procédé selon une des revendications 1 à 3, caractérisé en ce que l'alcool est choisi parmi le méthanol, l'éthanol et l'éthylèneglycol.

5. Procédé selon une des revendications 1 à 3, caractérisé en ce que le rapport molaire du 2,4-dinitro-5-fluoro-alkylbenzène l'hydroxyde de sodium ou à l'hydroxyde de potassium est de 1 : 1,4
